# EUROPEAN PATENT SPECIFICATION

(11) **EP 0 411 033 B1**
(45) Date of publication and mention of the grant of the patent: **07.09.1994**
(21) Application number: 89905303.7
(22) Date of filing: 27.04.1989
(51) Int. Cl.: C12N 15/29, A61K 37/02

(54) **STORAGE STABLE FORMULATIONS OF HOMOGENOUS DIMERIC M-CSF**
LAGERUNGSSTABILE ZUSAMMENSETZUNGEN VON HOMOGENEM DIMEREM M-CSF
FORMULATIONS STABLES PENDANT LE STOCKAGE DU M-CSF DIMERIQUE HOMOGENE

(30) Priority: 29.04.1988 US 187802; 23.05.1988 US 197499
(43) Date of publication of application: 06.02.1991
(73) Proprietor: GENETICS INSTITUTE, INC., Cambridge, Massachusetts 02140 (US)
(72) Inventor: MORRIS, Joseph, P., Chelmsford, MA 01824 (US); MORIN, Susan, Henry, Andover,MA 01810 (US); AMPHLETT, Godfrey, Boston, MA 02116 (US); SCHRIER, Jay, A., Andover, MA 01810 (US); WILLIAMS, Donald, F., Somerville, MA 02144 (US)
(74) Representative: Weinhold, Peter, Dr.
(86) International application number: US8901694
(87) International publication number: WO8910407

(56) References cited:
- WO-A-86/04607
- Science, vol. 235, 20 March 1987, G.G. Wong et al., pp. 1504-1508

## Description

Macrophage colony stimulating factor (M-CSF) is a regulatory glycoprotein that stimulates hematopoietic cell proliferation and differentiation. When M-CSF is applied to an in vitro colony stimulating assay it results in the formation of predominantly monocytic lineage type colonies.

M-CSF has significant therapeutic uses. For example, M-CSF may be used in activating mature white cells in cases of serious infection or as a therapeutic agent for naturally occurring or radiation-induced leukopenia. It may also be used for killing tumor cells either alone or by coadministering it with certain antibodies directed to tumor-associated antigens, as described in PCT/US88/00618 published as WO88/06452 on September 7, 1988. M-CSF may be administered to beneficially alter blood cholesterol levels.

Full-length M-CSF is described in Wong et al. Science, 235:1504-1508 (1987). The production of full length M-CSF by recombinant DNA techniques is described in Clark et al., PCT/US87/00835 published as WO 87/06954 on November 19, 1987. A cDNA sequence for a truncated variant M-CSF has been reported by Kawasaki et al., in Science, 230: 291-296 (1985). Recombinant production of that truncated version of M-CSF is described in PCT/US86/00238 published as WO 86/04607 on August 14, 1986. Variations on that truncated version, having deletions or substitutions of hydrophobic amino acids characteristic of a transmembrane region are described in EP 249,477, published December 16, 1987. Other recombinantly produced "short" and "long" forms of M-CSF, and muteins corresponding thereto, are described in Koths et al., PCT/US87/02679 published as WO 88/03173 on May 5, 1988. It is contemplated that the foregoing M-CSF polypeptides may be employed in this invention.

Suitable cells or cell lines for use in the recombinant production of M-CSF include mammalian cells, such as Chinese hamster ovary (CHO) cells, the monkey COS-1 cell line described in WO 87/06954, and the CV-1 cell line. Bacterial cells may also be used as expression systems; e.g., E. coli and B. subtilis. Additionally, many strains of yeast cells are available as host cells.

There is general agreement that the native M-CSF protein consist of two identical, heavily glycosylated subunits. This dimeric polypeptide has a complex quaternary structure maintained by both covalent and non-covalent binding.

Applicants have discovered that the integrity of this quaternary structure is compromised by surprisingly moderate pH and solvent effects, leading to the formation of multimeric molecular aggregates. "Multimeric molecular aggregates" is used herein to refer to high molecular weight species consisting of three or more whole subunits of M-CSF. Additionally, high molecular weight species also arise in M-CSF produced according to the method of Clark et al., International Publication Number WO 87/06954.

Applicants also have discovered that lyophilization and subsequent storage of M-CSF may also cause the formation and proliferation of multimeric molecular aggregates. These aggregates may be covalently or non-covalently bound to each other. The non-covalent, lyophilization-induced, aggregates have been shown to possess virtually no biological activity by the Wong Mouse Bone Marrow Assay, described in Wong, Science 235, supra.

Applicants have observed that multimeric molecular aggregrates of M-CSF are characterized by seriously compromised biological activity and hence therapeutic efficacy, and by higher immunogenicity and presumably toxicity. When mice were injected with these high molecular weight species, they produced a higher titer of antibodies to M-CSF than mice that were injected with the homogeneous dimeric M-CSF of this invention. This suggests that the high molecular weight species possess a higher toxicity than the homogeneous dimeric M-CSF. Further, high molecular weight M-CSF species have demonstrated as much as a ten-fold lower biological activity, relative to homogeneous dimeric M-CSF, as measured by the Wong Mouse Bone Marrow Assay.

To retain stability, activity, therapeutic efficacy, and most importantly, to reduce the risk of potential toxic side effects it is essential that pharmaceutical formulations of M-CSF are free of such high molecular weight species. Further, aggregation within protein solutions often leads to precipitation, which is deleterious because it can give rise to such recognized dangers as thrombosis, nonhomogeneity of dose, and clogged syringes.

The present invention also solves these problems by providing homogeneous dimeric M-CSF formulations that are stable to lyophilization and subsequent prolonged storage. M-CSF possesses greater therapeutic efficacy when the high molecular weight species (which have lower biological activity) are removed. The products of the present invention present less risk of immunogenic response.

As used in this application "homogeneous dimeric M-CSF" is defined as a protein that (a) is encoded by a DNA sequence that hybridizes under stringent conditions to the DNA sequence shown in Fig. 2 of Wong et al., and (b) exhibits an activity of at least about 0.4 x 10⁶ Dilution Units per milligram (DU/mg) in the Wong Mouse Bone Marrow Assay described in Wong et al. Science, supra, and (c) is substantially free from multimeric molecular aggregates having three or more M-CSF subunits. This covers, of course, not only the sequence which corresponds to the natural protein, and specifically shown in Fig. 2 of Wong et al., but also related sequences having point mutations, allelic variations, additions, and deletions which also meet the aforesaid requirements.

The homogeneous dimeric M-CSF can be obtained from partially purified crude preparations by utilizing gel filtration chromatography. Other steps will of course by employed to partially purify the crude dimeric M-CSF preparations from the supernatant, but their nature and order are not mandatory. In another aspect of our invention, the homogeneous dimeric M-CSF is prepared by passing a partially purified preparation of M-CSF over a gel filtration column.

In gel filtration chromatography, the bed is packed with gel medium or matrix made up of beads, which are porous to molecules of a specified size range. This size range is referred to as the fractionation range. A gel filtration matrix having a fractionation range (molecular weight) of about 10-1,500 kD may be used. Sephacryl S-300 (Pharmacia Biotechnology Products Catalog 86, 1986, Catalog # 17-0438-01) is such a matrix and is a currently preferred commercially available gel filtration matrix for purposes of this invention. Other functionally similar gel matrices, such as Sephacryl S-200 (Pharmacia Catalog # 17-0871-01), BioSil TSK-250 (Bio-Rad, Cat. # 125-0066), and TSK G-3000 SW (LKB, Cat. # 2135-360) may also be used.

Homogeneous dimeric M-CSF is characterized by a single peak when assayed by gel filtration chromatography using a gel filtration matrix having a fractionation range (molecular weight) of abouut 10-1,500 kD. This inventive substance has a specific activity of at least about 0.4 x 10⁶ DU/mg in the Wong Mouse Bone Marrow Assay. In a preferred specific embodiment, it has a specific activity of at least about 0.7 x 10⁶ DU/mg. For example, the M-CSF described in PCT/US87/00835 published as WO 87/06954 displays a broad band by nonreducing sodium dodecylsulfate polyacrylamide gel electrophoresis (SDS PAGE), due to variations in glycosylation, indicating an apparent molecular weight range of 70-90 kD. Among the reported versions of M-CSF, this 70-90 kD M-CSF most closely matches the characteristics of natural human M-CSF. This dimeric protein when purified to homogeneity in accordance with the present invention, has a specific activity which averages about 0.8 x 10⁶ DU/mg as measured by the Wong assay.

The products of this invention are formulated in lyophilized compositions comprising about 0.1-10% of M-CSF, about 0.5-20% of a pharmaceutically acceptable polyoxyethylenic non-ionic surfactant, about 40-75% glycine, about 15-40% sucrose, and up to about 25% of a pharmaceutically acceptable buffering agent. In a preferred embodiment, the products of this invention are formulated in a lyophilized composition comprising about 0.1-2% of M-CSF, about 0.1-9% of a pharmaceutically acceptable polyoxyethylenic non-ionic surfactant, about 65-75% glycine, about 17-21% sucrose, and about 4-7% of a pharmaceutically acceptable buffering agent. Percentages are by weight, unless otherwise specified. Upon reconstitution, these formulations have a pH of about 6. Protein analysis of the formulations exhibits only a single peak when assayed by gel filtration chromatography and a single molecular weight band by nonreducing SDS PAGE.

These formulations maintain M-CSF as a storage stable homogeneous dimer free of M-CSF aggregates. The term "stable" refers to substantial retention of the level of biological activity as determined by the production of predominantly macrophage-containing colonies in the Wong Mouse Bone Marrow Assay described in Wong et al., Science 235, supra.

The presence of non-covalently bound M-CSF aggregates is indicated by a gel filtration peak extraneous to the dimeric M-CSF peak in the gel filtration procedure described herein. The covalently bound aggregates are evidenced by a high molecular weight band on nonreducing SDS PAGE.

The preferred class of polyoxyethylenic non-ionic surfactants for use in the foregoing composition is the polysorbates. The preferred polysorbate is polysorbate 80. The preferred formulation has 0.005% polysorbate 80 prior to lyophilization. The poloxamers are another group of commercially available polyoxyethylenic non-ionic surfactants useful in this invention, particularly poloxamer 338. A preferred prelyophilized poloxamer formulation contains 0.05% of poloxamer 338.

The formulations of this invention further include glycine and sucrose and may be buffered to a pH of about 6 with a pharmaceutically acceptable buffering agent such as sodium citrate. Formulations having prelyophilized ranges of 0.25-0.75 M glycine, 0.5-3% sucrose, and 0-50 mM sodium citrate are exemplary. An exemplary preferred prelyophilized formulation contains 0.5 M glycine, 1% sucrose, and 10 mM sodium citrate.

The process of lyophilization is well known in the art. See Remington's Pharmaceutical Sciences, p.1538 (17th ed., 1985).

Homogeneous dimeric M-CSF and its storage stable, lyophilized, preferred formulation are especially suited for use in pharmaceutical formulations for parenteral administration. Such pharmaceutical formulations may comprise a therapeutically effective amount of homogeneous dimeric M-CSF in a parenterally acceptable vehicle. When parenterally administered, the therapeutic composition for use in this invention is in the form of a pyrogen-free, preferably aqueous isotonic solution. With respect to lyophilized formulations, parenteral administration may follow reconstitution, preferably with Water For Injection (WFI).

The therapeutically effective amount of M-CSF should be insufficient to cause a systemic toxic reaction, but sufficient to elicit the desired therapeutic response. The actual dosing regimen for such formulations will be determined by the attending physician considering various factors which modify the action of drugs, for example, the condition, body weight, sex and diet of the patient, time of administration, and the degree of onset of the disease.

An example which illustrates one method by which a homogeneous dimeric M-CSF may be obtained follows.

### EXAMPLE 1

Full-length M-CSF was produced in CHO cells in accordance with the method of Clark et al., International Publication Number WO 87/06954. Crude protein was harvested from conditioned medium and applied to a membrane type anion exchange column (QAE Zeta Prep Anion Exchange disk [LKB Produkter AB]). The M-CSF bound to this disk, as did a host of other negatively charged proteins. The column was washed and the M-CSF eluted with a buffer containing 0.5 M NaCl.

The eluate of the QAE Zeta Prep disk was prepared for chromatography on immobilized lentil lectin (Lentil Lectin-Sepharose [Pharmacia]) by the addition of Polysorbate 20, to a concentration of 0.05% (V/V). The product eluted from the column with a buffer containing methyl-α,D-mannopyranoside

The eluate of the immobilized lentil lectin column was then prepared for application onto a high resolution anion exchange column (Mono Q resin [Pharmacia]). The product fraction from the lentil lectin step was acidified to pH 5.8 with 200mM MES buffer and loaded onto the Mono Q column. The M-CSF eluted off the column with a linear gradient of from 0.05 M NaCl to 0.3 M NaCl. Certain high molecular weight species of M-CSF were present in the latter fractions of the elution, which were excluded from the M-CSF pool.

The pooled product fractions from the Mono Q chromatography step were acidified prior to loading it onto a C₄ (Vydac) reversed phase HPLC column. The C₄ column was equilibrated with a buffer of 27% aqueous acetonitrile and 0.1% TFA. The column was then washed and the M-CSF eluted with a linear gradient of from 40.5% to 58.5% aqueous acetonitrile/0.1% TFA. The M-CSF containing fractions were pooled.

Following the C₄ reversed phase HPLC step, the M-CSF containing fractions were concentrated on a DEAE-Sepharose anion exchange column. The M-CSF pool was diluted 5 fold with DEAE equilibration buffer and loaded onto the DEAE column. The M-CSF eluted with a buffer containing 0.5M NaCl.

The DEAE-Sepharose eluate was loaded directly onto a Sephacryl S-300 [Pharmacia] gel filtration column having a column size of 90 cm x 9 cm, and eluted with 20mM sodium citrate, 50 mM NaCl, pH 6.0, at a flow rate of 2 mL/minute. The M-CSF was eluted in an isocratic mode with the equilibration buffer. The resulting chromatogram displayed two well resolved peaks. The peak corresponding to homogeneous dimeric (70-90 kD) M-CSF appeared behind that of the faster migrating, high molecular weight species. The fractions corresponding to homogeneous dimeric M-CSF were pooled and chromatographed for analytical purposes through a 600 mm x 7.5 mm Bio-Rad BioSil 250 column eluting with a buffer of 50mM Na₂SO₄, 20mM Na₂HPO₄, pH 6.8, and 0.02% sodium azide at a flow rate of 1 mL/minute giving a chromatogram displaying a single peak. The pooled homogeneous dimeric M-CSF was frozen and stored at -80°C.

While determination of effective column conditions and elution buffers for gel filtration, generally, is within the skill of the art, Applicants' currently preferred conditions for purposes of this invention are those described above.

An example which illustrates a storage stable, lyophilized formulation of M-CSF follows:

### EXAMPLE 2.

Full-length M-CSF was produced in CHO cells in accordance with the method of Clark et al., International Publication Number WO 87/06954. Homogeneous dimeric M-CSF was obtained in accordance with the method of Example 1.

A 200 ml solution of 0.125 mg/ml M-CSF, 10 mM sodium citrate, 0.5 M glycine, 1% sucrose, and 0.005% polysorbate 80 at pH 6 was filtered through a 0.2 µm filter (Durapore). The filtered solution, in 2 ml aliquots, was dispensed into 100 10 ml vials suitable for lyophilization. The vials were loaded into the lyophilizer and frozen at -40°C for one hour. The condenser was chilled to -70°C, the product temperature was raised to -37°C for 1/2 hour, and the chamber pressure was reduced to 50 mTorr. Pressure was maintained by a dry nitrogen bleed. The shelf temperature was raised such that the product temperature was raised to -35°C to commence the primary drying. Primary drying was considered complete when the product temperature and the shelf temperature came into equilibrium. The shelf-surface temperature was then increased to +20°C at a rate such that the shelf-surface temperature and the product temperature differed by no more than 10°C. When the partial pressure of water vapor was less than 5 mTorr, the system was backfilled with dry nitrogen and the product vials were stoppered.

A representative lyophilized sample, containing 0.2% M-CSF, 0.1% polysorbate 80, 74.1% glycine, 19.7% sucrose and 5.8 % sodium citrate was then reconstituted to 2 ml with WFI and analyzed by gel filtration and nonreducing SDS PAGE. The gel filtration chromatogram showed a single peak and the SDS PAGE analysis showed a single molecular weight band at about 80 kD.

## Claims

1. A storage stable lyophilized M-CSF formulation of a homogeneous dimeric M-CSF, which is characterized by a single peak when assayed by gel filtration chromatography using a gel filtration matrix having a fractionation range of about 10-1500 kD, comprising about 0.1-10% by weight of M-CSF, about 0.5-20% by weight of a pharmaceutically acceptable polyoxyethylenic non-ionic surfactant, about 40-75% by weight glycine, about 15-40% by weight sucrose, and to about 25% by weight of a pharmaceutically acceptable buffering agent, having a pH of about 6 upon reconstitution.

2. An M-CSF formulation according to claim 1 comprising about 0.1-2.0% by weight of M-CSF, about 0.1-9% by weight of a pharmaceutically acceptable polyoxyethylenic non-ionic surfactant, about 65-75% by weight glycine, about 17-21% by weight sucrose, and about 4-7% by weight of a pharmaceutically acceptable buffering agent, having a pH of about 6 upon reconstitution.

3. An M-CSF formulation according to claim 1 or 2, wherein the M-CSF has a specific activity of at least about 0.4 x 10⁶ DU/mg in the Wong Mouse Bone Marrow Assay.

4. An M-CSF formulation according to any one of claims 1 to 3, wherein the M-CSF is characterized by a molecular weight of about 70-90 kD by nonreducing SDS PAGE.

5. An M-CSF formulation according to any one of claims 1 to 4, wherein said surfactant is polysorbate 80.

6. Use of an M-CSF formulation according to any one of claims 1 to 5 for the preparation of a pharmaceutical formulation for parenteral administration.

## Patentansprüche

1. Lagerstabile lyophilisierte M-CSF-Formulierung eines homogenen dimeren M-CSF, der bei der Analyse mittels Gelfiltration unter Verwendung einer Gelfiltrationsmatrix mit einem Auftrennungsbereich von 10-1500 kD durch einen einzelnen Peak gekennzeichnet ist, die ungefähr 0,1-10 Gew.-% M-CSF, ungefähr 0,5-20 Gew.-% eines pharmazeutisch annehmbaren polyoxyethylenischen, nichtionischen oberflächenaktiven Mittels, ungefähr 40-75 Gew.-% Glycin, ungefähr 15-40 Gew.-% Sucrose und bis ungefähr 25 Gew.-% eines pharmazeutisch annehmbaren Pufferungsmittels umfaßt und nach Rekonstitution einen pH von ungefähr 6 hat.

2. M-CSF-Formulierung nach Anspruch 1, die ungefähr 0,1-2,0 Gew.-% M-CSF, ungefähr 0,1-9 Gew.-% eines pharmazeutisch annehmbaren polyoxyethylenischen, nichtionischen oberflächenaktiven Mittels, ungefähr 65-75 Gew.-% Glycin, ungefähr 17-21 Gew.-% Sucrose und ungefähr 4-7 Gew.-% eines pharmazeutisch annehmbaren Pufferungsmittels umfaßt und nach Rekonstitution einen pH von ungefähr 6 hat.

3. M-CSF-Formulierung nach Anspruch 1 oder 2, worin der M-CSF im Mäuse-Knochenmarkstest nach Wong eine spezifische Aktivität von wenigstens ungefähr 0,4 x 10⁶ DU/mg hat.

4. M-CSF-Formulierung nach irgendeinem der Ansprüche 1 bis 3, worin der M-CSF durch ein Molekulargewicht von ungefähr 70-90 kD bei nichtreduzierender SDS-PAGE gekennzeichnet ist.

5. M-CSF-Formulierung nach irgendeinem der Ansprüche 1 bis 4, worin das oberflächenaktive Mittel Polysorbat (Polyoxyethylensorbitanester) 80 ist.

6. Verwendung einer M-CSF-Formulierung nach irgendeinem der Ansprüche 1 bis 5 zur Herstellung einer pharmazeutischen Formulierung zur parenteralen Verabreichung.

## Revendications

1. Formulation de M-CSF lyophilisée stable au stockage d'un M-CSF dimère homogène qui est caractérisé par un pic unique lors de l'analyse par chromatographie par filtration sur gel avec une matrice de filtration sur gel ayant un domaine de fractionnement d'environ 10-1500 kD, comprenant environ 0,1-10 % en masse de M-CSF, environ 0,5-20% en masse d'un agent tensio-actif polyoxyéthylénique non ionique pharmaceutiquement acceptable, environ 40-75 % en masse de glycine, environ 15-40 % en masse de sucrose, et jusqu'à environ 25 % en masse d'un tampon pharmaceutiquement acceptable, ayant un pH d'environ 6 après reconstitution.

2. Formulation de M-CSF selon la revendication 1, comprenant environ 0,1-2,0 % en masse de M-CSF, environ 0,1-9 % en masse d'un agent tensio-actif polyoxyéthylénique non ionique pharmaceutiquement acceptable, environ 65-75 % en masse de glycine, environ 17-21 % en masse de sucrose, et environ 4-7 % en masse d'un tampon pharmaceutiquement acceptable, ayant un pH d'environ 6 après reconstitution.

3. Formulation de M-CSF selon la revendication 1 ou 2, dans laquelle le M-CSF a une activité spécifique d'au moins environ 0,4 x 10⁶ UD/mg dans l'essai sur la moelle osseuse de souris de Wong.

4. Formulation de M-CSF selon l'une quelconque des revendications 1 à 3, dans laquelle le M-CSF est caractérisé par une masse moléculaire d'environ 70-90 kD mesurée par électrophorèse sur gel de polyacrylamide-dodécylsulfate de sodium (SDS-PAGE) non réductrice.

5. Formulation de M-CSF selon l'une quelconque des revendications 1 à 4, dans laquelle ledit agent tensio-actif est le polysorbate 80.

6. Utilisation d'une formulation de M-CSF selon l'une quelconque des revendications 1 à 5 pour la préparation d'une formulation pharmaceutique pour l'administration parentérale.
